Europäisches Patentamt

European Patent Office

Office européen des brevets

(19)

(11) Veröffentlichungsnummer: **0 012 184**
**B1**

(12) **EUROPÄISCHE PATENTSCHRIFT**

(45) Veröffentlichungstag der Patentschrift:
30.12.81

(51) Int. Cl.³: **C 12 Q 1/54,** G 01 N 33/66

(21) Anmeldenummer: 79104098.3

(22) Anmeldetag: 23.10.79

(54) Hämolysierlösung, Verwendung einer solchen für die Bestimmung von Glucose im Blut, und Hämolyseverfahren.

(30) Priorität: 22.11.78 DE 2850603

(43) Veröffentlichungstag der Anmeldung:
25.06.80 Patentblatt 80/13

(45) Bekanntmachung des Hinweises auf die Patenterteilung:
30.12.81 Patentblatt 81/52

(84) Benannte Vertragsstaaten:
AT BE CH DE FR GB IT NL SE

(56) Entgegenhaltungen:
US-A-3 634 290
US-A-3 953 295

(73) Patentinhaber: Merck Patent Gesellschaft mit
beschränkter Haftung, Frankfurter Strasse 250,
D-6100 Darmstadt (DE)

(72) Erfinder: Vormbrock, Rolf, Dr., Grundstrasse 17,
D-6100 Darmstadt (DE)
Erfinder: Helger, Roland, Dr., Ludwigshöhstrasse 85,
D-6100 Darmstadt (DE)

Hämolysierlösung, Verwendung einer solchen für die Bestimmung von Glucose im Blut
und Hämolyseverfahren

Die vorliegende Erfindung betrifft eine Hämolysierlösung sowie ein Verfahren zur Hämolyse von Blut und zur Stabilisierung der Glucosekonzentration in hämolysiertem Blut.

Die Bestimmung der Glucosekonzentration im Vollblut wird wegen des geringen Arbeitsaufwandes einer Bestimmung in Plasma oder Serum vorgezogen. Ein weiterer Vorteil bei der Verwendung von Vollblut besteht darin, dass bereits eine sehr geringe Menge Blut (z.B. 20 µl) für eine Bestimmung ausreicht. In der entnommenen Blutprobe bewirken jedoch die in den Erythrozyten enthaltenen Enzyme einen glykolytischen Abbau der vorhandenen Glucose. Aus diesem Grund muss die Glucosebestimmung entweder unverzüglich nach der Blutentnahme durchgeführt werden oder die Glykolyse muss durch geeignete Methoden inhibiert werden. Die Inhibierung kann sowohl in isotonischer Lösung ohne Hämolyse erfolgen als auch nach der Hämolyse der Probe, die z.B. durch osmotischen Schock oder mittels Digitonin hervorgerufen werden kann.

In der Literatur sind bereits eine Reihe von Inhibitoren der Glykolyse beschrieben worden, z.B. Fluoride, Halogenacetate, N-Alkylmaleinimide, Essigsäure usw. Der Hauptnachteil der bekannten, diese Inhibitoren enthaltenden Hämolysierlösungen für die Glucosebestimmung in Vollblut besteht darin, dass diese Lösungen entweder nur eine begrenzte Zeit stabil sind oder die Glykolyse nicht vollständig inhibieren. So beträgt z.B. die Haltbarkeit der Hämolysierlösung nach der DE-AS 18 13 848 nur etwa 3–4 Wochen, d.h., diese Lösungen müssen in geringen Zeitabständen frisch angesetzt werden, um sicher zu sein, dass sie noch voll funktionsfähig sind.

Der Erfindung liegt die Aufgabe zugrunde, eine Hämolysierlösung für die Glucosebestimmung im Vollblut zur Verfügung zu stellen, die gut hämolysiert, die Glykolyse hemmt und praktisch unbegrenzt haltbar ist.

Erfindungsgemäss wurde diese Aufgabe durch eine Hämolysierlösung gelöst, die neben einem Puffer und Natriumchlorid einen Chelatbildner und ein Tensid enthält.

Gegenstand der Erfindung ist demnach eine Hämolysierlösung für die Glucosebestimmung im Blut, die im wesentlichen einen Puffer und Natriumchlorid enthält und die gekennzeichnet ist durch einen Gehalt an mindestens einem Chelatbildner und mindestens einem Tensid.

Ferner umfasst der Gegenstand der Erfindung ein Verfahren zur Hämolyse von Blut und zur Stabilisierung der enthaltenen Glucosekonzentration, das dadurch gekennzeichnet ist, dass man Vollblut mit einer wässrigen Hämolysierlösung behandelt, die im wesentlichen Puffer, Natriumchlorid, Chelatbildner und Tensid enthält.

Ein weiterer Gegenstand der Erfindung besteht in der Verwendung einer Kombination aus mindestens einem Chelatbildner und mindestens einem Tensid in Hämolysierlösungen für die Glucosebestimmung im Blut.

Überraschenderweise hat sich gezeigt, dass die Kombination aus Chelatbildner und Tensid eine vollständige Inhibierung der Glykolyse im Hämolysat bewirkt und ausserdem eine stabile Hämolysierlösung erhalten wird. Chelatbildner wie Äthylendiamintetraacetat, die durch Komplexierung zweiwertiger Metallkationen eine Reihe von Enzymen der Glykolyse zu inhibieren vermögen, sind bisher nicht als Stabilisatoren in Hämolysierlösungen verwendet worden, weil Chelatbildner allein keine effektive Hemmung der Glykolyse im Blut bewirken; bei einer Äthylendiamintetraacetatkonzentration von z.B. 1 g/l wird die im Blut enthaltene Glucose vielmehr abgebaut. Die Glucosekonzentration im mit der erfindungsgemässen Hämolysierlösung hergestellten Hämolysat ist dagegen nach 12 Tagen immer noch unverändert. Ein zusätzlicher Vorteil der erfindungsgemässen Hämolysierlösung besteht darin, dass im Hämolysat keine Trübung auftritt.

Als Tenside eignen sich ionische Tenside, wie Natriumdodecylsulfat, Cetyltrimethylammoniumbromid, Laurylarkosin oder Tauroglycocholat, vor allem nichtionische Tenside, vorzugsweise Alkylphenolpolyglykoläther.

Geeignete Chelatbildner sind z.B. Äthylendiamintetraacetat, Nitrilotriacetat, Cyclohexylen-(1,2)-dinitrilotetraacetat, Diäthylentriaminpentaacetat, Bis-(aminoäthyl)-glykoläther-N,N,N',N'-tetraacetat, vorzugsweise Äthylendiamintetraacetat.

In der Hämolysierlösung können jeweils auch Kombinationen der Chelatbildner und Tenside eingesetzt werden.

Die erfindungsgemässe Hämolysierlösung enthält die Tenside in einer Konzentration von 1–4 g/l, vorzugsweise 2 g/l; die Chelatbildner sind in einer Konzentration von 0,5 bis 2 g/l, vorzugsweise 1 g/l enthalten.

Die Hämolysierlösung enthält darüber hinaus noch mindestens einen Puffer und Natriumchlorid. Der Puffer dient dazu, den pH-Wert der Hämolysierlösung in einem für die Glucosebestimmung geeigneten Bereich von etwa 6–8 einzustellen. Als geeignete Puffer haben sich z.B. Phosphat-, Tris-, Triäthanolamin- oder Imidazolpuffer in einer Konzentration von 50–500 mmol/l erwiesen, bevorzugt wird ein Phosphatpuffer vom pH-Wert 7,6 in einer Konzentration von 120 mmol/l verwendet. Die Natriumchloridkonzentration sollte grösser als 0,8 mol/l sein, vorzugsweise etwa 1 mol/l.

Zur Vermeidung mikrobieller Kontamination können noch 5–120 mmol/l eines Alkaliazids zugesetzt werden, vorzugsweise 14 mmol/l Natriumazid.

Die Hämolyse von Vollblut unter gleichzeitiger Stabilisierung der enthaltenen Glucosekonzentration erfolgt in der Weise, dass man das zu untersuchende Vollblut mit der erfindungsgemässen wässrigen Hämolysierlösung aufnimmt und anschliessend oder aber erst nach einigen Tagen mit dieser Lösung die enzymatische Glucosebestimmung nach den üblichen Methoden vornimmt.

Die Erfindung wird durch die folgenden Beispiele näher erläutert.

Beispiel 1

20 µl Blut werden unmittelbar nach der Blutabnahme in 2 ml einer Hämolysierlösung aufgenommen, die folgende Zusammensetzung hat:

120 mmol/l Phosphatpuffer, pH 7,6
1 mol/l Natriumchlorid
14 mmol/l Natriumazid
1 g/l Äthylendiamintetraacetat und
2 g/l Polyoxyäthylen-10-alkylphenoläther
(Lutensol[R] AP 10).

1 ml dieser Lösung wird in eine Küvette pipettiert und mit 2 ml Pufferlösung folgender Zusammensetzung versetzt:

120 mmol/l Phosphatpuffer, pH 7,6
150 mmol/l Natriumchlorid
14 mmol/l Natriumazid
2,8 mmol/l Äthylendiamintetraacetat
3,6 mmol/l Nicotinamidadenindinucleotid und
150 U/l Mutarotase.

Die Extinktion der Lösung wird bei einer Wellenlänge von 340 nm oder mit Filter Hg 334 oder Hg 365 gemessen, dann wird durch Zugabe von 20 µl Enzymlösung, die 500 kU/l Glucosedehydrogenase enthält, die Reaktion gestartet. Nach etwa 5 Minuten ist die Reaktion beendet und die Extinktion wird abgelesen. Aus der Extinktionsdifferenz $\Delta E$ errechnet sich die Glucosekonzentration der untersuchten Probe nach der Gleichung:

Glucosekonzentration $c = \Delta E_{334} \times 889{,}2$ mg/dl.

Die Glucosekonzentration bleibt über Wochen konstant, die Hämolysierlösung ist unbegrenzt stabil.

Beispiel 2

Analog Beispiel 1 wurde die Glucosekonzentration in einer hämolysierten Blutprobe bestimmt, wobei das Hämolysat in 6 Teile geteilt wurde, die jeweils im Abstand von einigen Tagen gemessen und ausgewertet wurden. Zum Vergleich wurden Blutproben gemessen, die mit einer Hämolysierlösung behandelt waren, die Natriumfluorid an Stelle der Kombination aus Chelatbildner und Tensid enthielt.

Die nachstehende Tabelle gibt die Glucosekonzentration der verschiedenen Hämolysate in % des Anfangswertes wieder. Die Werte wurden mit Proben erhalten, die 70 mg/dl Glucose enthielten.

| Stabilisator | Aufbewahrungszeit (Tage) | | | | | |
|---|---|---|---|---|---|---|
| | 1 | 2 | 5 | 7 | 9 | 12 |
| EDTA 1,0 g/l Tensid 2,0 g/l | 102 | 103 | 103 | 102 | 102 | 103 |
| NaF 0,5 g/l | 98,7 | 94,1 | 88,3 | 86,3 | 86,3 | 78,5 |

Aus der Tabelle geht hervor, dass die Glucosekonzentration in den mit der erfindungsgemässen Hämolysierlösung hergestellten Hämolysaten im Versuchszeitraum konstant bleibt, während in den Natriumfluorid enthaltenden Hämolysaten die Glucosekonzentration ständig abnimmt.

**Patentansprüche**

1. Hämolysierlösung für die Glucosebestimmung im Blut, im wesentlichen enthaltend einen Puffer und Natriumchlorid, gekennzeichnet durch einen Gehalt an mindestens einem Chelatbildner und mindestens einem Tensid.

2. Hämolysierlösung nach Anspruch 1, dadurch gekennzeichnet, dass als Tenside nichtionische Tenside enthalten sind.

3. Hämolysierlösung nach den Ansprüchen 1 und 2, dadurch gekennzeichnet, dass als Tenside Alkylphenolpolyglykoläther enthalten sind.

4. Hämolysierlösung nach den Ansprüchen 1 bis 3, dadurch gekennzeichnet, dass als Chelatbildner Äthylendiamintetraacetat, Nitrilotriacetat, Cyclohexylen-(1,2)-dinitrilotetraacetat, Diäthylentriaminpentaacetat und/oder Bis-(aminoäthyl)-glykoläther-N,N,N',N'-tetraacetat enthalten sind.

5. Hämolysierlösung nach den Ansprüchen 1 bis 4, dadurch gekennzeichnet, dass die Tenside in einer Konzentration von 1 bis 4 g/l enthalten sind.

6. Hämolysierlösung nach den Ansprüchen 1 bis 5, dadurch gekennzeichnet, dass die Chelatbildner in einer Konzentration von 0,5 bis 2 g/l enthalten sind.

7. Hämolysierlösung nach den Ansprüchen 1 bis 6, dadurch gekennzeichnet, dass zusätzlich Natriumazid enthalten ist.

8. Verfahren zur Hämolyse von Blut und zur Stabilisierung der enthaltenen Glucosekonzentration, dadurch gekennzeichnet, dass man Vollblut mit einer wässrigen Hämolysierlösung behandelt, die im wesentlichen Puffer, Natriumchlorid, Chelatbildner und Tensid enthält.

9. Verwendung einer Kombination aus mindestens einem Chelatbildner und mindestens einem Tensid in Hämolysierlösungen für die Glucosebestimmung im Blut.

**Revendications**

1. Solution hémolysante pour la détermination du glucose dans le sang, contenant essentiellement un tampon et du chlorure de sodium et caractérisée en ce qu'elle contient au moins un agent chélatant et au moins un agent tensio-actif.

2. Solution hémolysante selon la revendication 1, caractérisée en ce qu'elle contient en tant qu'agents tensio-actifs des agents tensio-actifs non-ioniques.

3. Solution hémolysante selon les revendications 1 et 2, caractérisée en ce qu'elle contient en tant qu'agents tensio-actifs des éthers de polyglycol d'alkylphénols.

4. Solution hémolysante selon les revendications 1 à 3, caractérisée en ce qu'elle contient en tant qu'agents chélatants de l'éthylène-diamino-cétate, du nitrilotriacétate, du cyclohexylène-(1,2)-dinitrilotétracétate, du diéthylène-triaminopenta-cétate et/ou du N,N,N',N'-tétracétate de l'éther bis-(aminoéthylique) du glycol.

5. Solution hémolysante selon les revendications 1 à 4, caractérisée en ce qu'elle contient les agents tensio-actifs à une concentration de 1 à 4 g/l.

6. Solution hémolysante selon les revendications 1 à 5, caractérisée en ce qu'elle contient les agents chélatants à une concentration de 0,5 à 2 g/l.

7. Solution hémolysante selon les revendications 1 à 6, caractérisée en ce qu'elle contient en outre de l'azide de sodium.

8. Procédé pour hémolyser le sang et stabiliser sa concentration en glucose, caractérisé en ce que l'on traite le sang entier par une solution aqueuse hémolysante contenant essentiellement un tampon, du chlorure de sodium, un agent chélatant et un agent tensio-actif.

9. Utilisation d'une combinaison d'au moins un agent chélatant et d'au moins un agent tensio-actif dans des solutions hémolysantes pour la détermination du glucose dans le sang.

**Claims**

1. Haemolysing solution, for the determination of glucose in blood, essentially containing a buffer and sodium chloride, characterised in that it contains at least one chelating agent and at least one surface-active agent.

2. Haemolysing solution according to Claim 1, characterised in that it contains non-ionic surface-active agents as the surface-active agents.

3. Haemolysing solution according to Claims 1 and 2, characterised in that it contains alkylphenol polyglycol ethers as the surface-active agents.

4. Haemolysing solution according to Claims 1 to 3, characterised in that it contains ethylenediaminetetraacetate, nitrilotriacetate, cyclohexylene-1,2-dinitrilotetraacetate, diethylenetriaminepentaacetate and/or bis-(aminoethyl)-glycol ether-N,N,N',N'-tetraacetate as the chelating agents.

5. Haemolysing solution according to Claims 1 to 4, characterised in that it contains the surface-active agents in a concentration of 1 to 4 g/l.

6. Haemolysing solution according to Claims 1 to 5, characterised in that in contains the chelating agents in a concentration of 0.5 to 2 g/l.

7. Haemolysing solution according to Claims 1 to 6, characterised in that it additionally contains sodium azide.

8. Process for the haemolysis of blood and for stabilising the concentration of glucose therein, characterised in that complete blood is treated with an aqueous haemolysing solution which essentially contains a buffer, sodium chloride, a chelating agent and a surface-active agent.

9. Use of a combination of at least one chelating agent and at least one surface-active agent in haemolysing solutions for the determination of glucose in blood.